# EUROPEAN PATENT APPLICATION

(11) **EP 4 664 472 A1**
(43) Date of publication of application: **17.12.2025**
(21) Application number: 24204051.7
(22) Date of filing: 01.10.2024
(51) Int. Cl.: G16H 30/40, G16H 50/20, A61B 5/00, G06N 3/045, G06T 7/00, G06V 10/764, G06V 10/82

(54) **HEALTH STATUS EVALUATION METHOD AND SCANNER USING THE SAME**

(30) Priority: 14.06.2024 TW 113122013
(71) Applicant: Quanta Computer Inc., Taoyuan City 333 (TW)
(72) Inventor: CHANG, Chia-Yuan, 333 Taoyuan City (TW); CHENG, Kai-Ju, 333 Taoyuan City (TW); LEE, Hao-Ping, 333 Taoyuan City (TW); CHEN, Shao-Ang, 333 Taoyuan City (TW); CHEN, Kuan-Chung, 333 Taoyuan City (TW); CHEN, Yu-Hsun, 333 Taoyuan City (TW)
(74) Representative: Michalski Hüttermann & Partner Patentanwälte mbB

(57) **Abstract**

A health status evaluation method is provided. The method includes: receiving a body-part image; classifying the body-part image; selecting a processing model suitable for evaluating the body-part image; using the selected processing model to perform a health status evaluation on the body part corresponding to the body-part image; and integrating and outputting the health status evaluation results of the processing model.

## Description

### BACKGROUND OF THE INVENTION

### Field of the Invention

The present invention relates to a scanning device, and in particular it relates to a scanning device for scanning images of body parts and a health status evaluation method based on the body-part images.

### Description of the Related Art

Recent years have seen a trend wherein deep learning, machine learning, and artificial intelligence (AI) are being employed in the medical field. Various excellent models have been developed and used in different medical environments. The aforementioned models are usually operated independently. In practical applications, medical personnel still need to judge and select the corresponding models one by one, and then comprehensively analyze the inference results of each model to provide a comprehensive evaluation. As a result, there is still room for improvement in the efficiency of medical processes and the use of valuable time and resources.

Based on the description above, there is a need to provide a new evaluation mechanism to achieve faster medical decisions and more accurate evaluation solutions through automated classification and model selection.

### BRIEF SUMMARY OF THE INVENTION

The present invention proposes a new health status evaluation method and a scanning device using the same. Through image classification and object detection technology, the present invention first pre-classifies (pre-groups) bod-part images, then automatically selects appropriate processing models based on the classification results to evaluate the health status of body parts. Finally, the evaluation results of all processing models are integrated and a report of overall results is given.

According to one embodiment of the present invention, a health status evaluation method, includes: obtaining a body-part image from an imaging device; classifying the body-part image; selecting a processing model suitable for evaluating the body-part image based on the classification result; using the selected processing model to perform health status evaluation on the body part corresponding to the body-part image; and integrating and outputting the health status evaluation result of the processing model.

According to another embodiment of the present invention, a scanning device, includes: an imaging device, an orientation sensor, and a controller. The imaging device is configured to obtain a body-part image. The orientation sensor is configured to obtain orientation data related to the imaging device. The controller is configured to execute the steps of: receiving the body-part image from the imaging device; classifying the body-part image; selecting a processing model suitable for evaluating the body-part image based on the classification result; using the selected processing model to perform health status evaluation on the body part corresponding to the body-part image; and integrating and outputting the health status evaluation result of the processing model.

### BRIEF DESCRIPTION OF THE DRAWINGS

The present invention can be more fully understood by reading the subsequent detailed description and examples with references made to the accompanying drawings, wherein:
FIG. 1 shows a schematic configuration of a scanning device according to a first embodiment of the present invention.
FIG. 2 shows a schematic configuration of a scanning device according to a second example of the present invention.
FIG. 3 shows a schematic diagram of the distribution of teeth in the oral cavity.
FIG. 4A shows an exemplary image of teeth in oral cavity.
FIG. 4B and FIG. 4C show schematic diagrams of tooth images classified to different regions.
FIG. 5 is a schematic diagram showing the execution of object detection according to the present invention.
FIG. 6 shows a schematic diagram of annotating properties with text after executing the object detection.
FIG. 7 shows a schematic diagram of identifying canine teeth, incisor teeth and dental plaque during object detection.

### DETAILED DESCRIPTION OF THE INVENTION

The following description is made for the purpose of illustrating the general principles of the invention and should not be taken in a limiting sense. The scope of the invention is best determined by reference to the appended claims.

### [First embodiment]

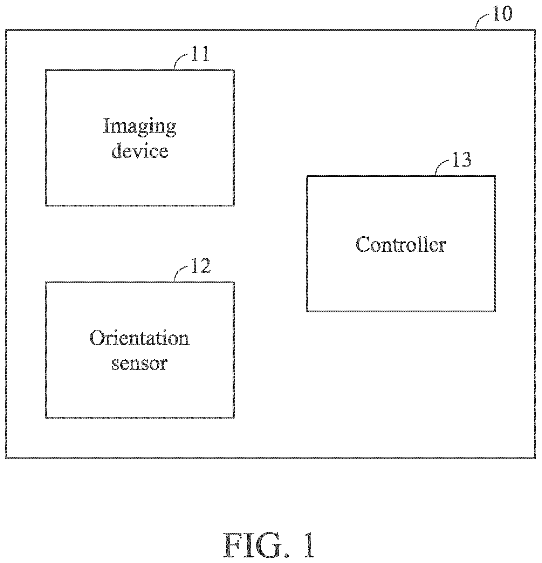

FIG. 1 shows a schematic configuration of a scanning device 10 according to a first embodiment of the present invention. The scanning device 10 of the first embodiment of the present invention is, for example, a body scanner, which can be used to scan images of various parts (locations) of the body, such as facial images, hand images, or images of other parts (location).

The scanning device 10 in FIG. 1 has an imaging device 11, an orientation sensor 12, and a controller 13. The imaging device 11 is, for example, a camera or any other device that can capture images. The orientation sensor 12 may be provided independently from the camera, or may be integrated into the camera. The orientation sensor 12 and the imaging device 11 (camera) work together. When the imaging device 11 captures an image, the orientation sensor 12 also detects the angle, position, etc. of the imaging device 11 when capturing the image, and records it as orientation data.

In the first embodiment, the controller 13 of the scanning device 10 is configured to perform the following operations. First, the controller 13 receives body-part images, in this embodiment, for example facial images, from the imaging device 11. The facial images may include images of eyes, ears, nose, or mouth, etc. Therefore, the controller 13 further classifies (or groups) the received body-part images to identify and classify the received body-part images to the images at the location of eyes, ears, nose, or mouth.

The controller 13 further selects a processing model suitable for evaluating the classified body-part images based on the classification results. For example, based on the classification results, if there are images of eyes, ears, nose, or mouth at the same time, the controller 13 will call corresponding processing models designed for eyes, ears, nose, and mouth, to analyze and evaluate the images at the locations of eyes, ears, nose, or mouth.

Similarly, if the body-part image received from the imaging device 11 is, for example, a hand image, the controller 13 will further classify the received hand image to a finger image, nail image, or palm image at different location of hand. Then, the controller 13 executes the corresponding processing model to evaluate the health status of the finger part, nail part, and palm part.

In the first embodiment, the controller 13 classifies the received body-part images, for example, by executing a simple computer graphics recognition program, or by executing a deep learning classification model that has completed training, to identify and classify the body-part images. The deep learning classification model can be trained using various body-part images which have been pre-labeled feature points for specific body parts (such as eyes, ears, nose, etc. in facial images; fingers, nails, palms, etc. in hand images) for deep learning training.

Here, the processing model is an evaluation application tool or program suitable for the body part to be evaluated, such as the eyes of the face or the fingers of the hand. After the controller 13 executes the processing model, the controller 13 can link to the corresponding medical database to obtain the data of the corresponding body parts to evaluate the health status, and integrates and generates an evaluation report.

### [Second embodiment]

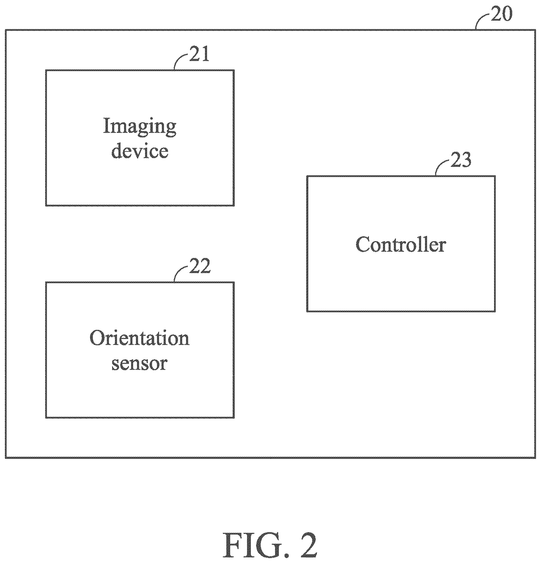

FIG. 2 shows a schematic configuration of a scanning device 20 according to a second example of the present invention. The scanning device 20 of the second embodiment according to the present invention is, for example, an oral scanner, which scans images of the oral cavity, and obtains for example teeth images, gum images, dental plaque images, etc. in the oral cavity.

In FIG. 2, the scanning device 20 has an imaging device 21, an orientation sensor 22, and a controller 23. As described in the first embodiment, the imaging device 21 is, for example, a camera or any other device that can capture images. The orientation sensor 22 may be provided independently from the camera, or may be integrated into the camera. The orientation sensor 22 and the imaging device 21 (camera) jointly work. When the imaging device 21 captures an image, the orientation sensor 22 also detects the angle, position, etc. of the imaging device 21 when capturing the image, and records it as orientation data.

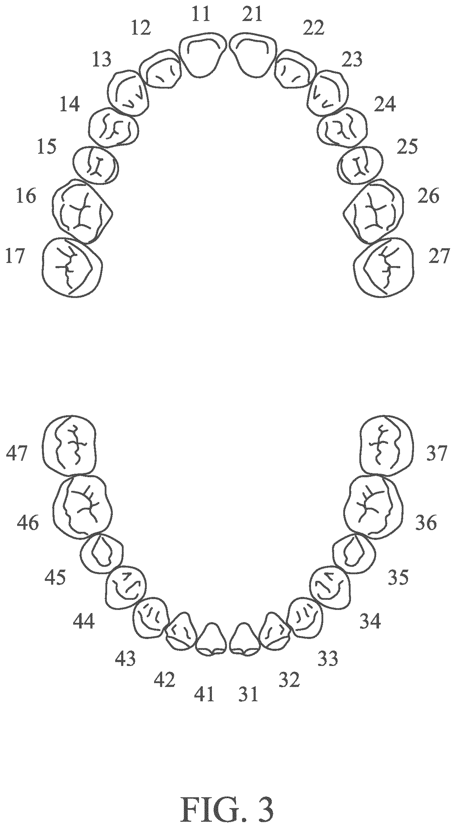

FIG. 3 shows a schematic diagram of the distribution of teeth in the oral cavity. In FIG. 3, two numbers are assigned to each tooth. The previous number represents the quadrant where the tooth is located, and the latter number represents the sequence. Therefore, according to the positions of the teeth in the oral cavity, they can be roughly divided into the "upper right quadrant corresponding to the previous number 2", "upper left quadrant corresponding to the previous number 1", "lower right quadrant corresponding to the previous number 3", and "lower left quadrant corresponding to the previous number 4". Here, the four quadrants are examples and are not intended to limit the invention. In addition, incisor teeth and canine teeth generally have a front surface (outer surface) and an inner surface (inside surface). In addition to the front and inner surfaces, molar teeth also have chewing surfaces. Therefore, the markings on the teeth in FIG. 3 can further indicate that they are in the front, inner or chewing surface regions.

Referring to FIG. 2 again, the scanning device 20 obtains the oral cavity image from the imaging device 21. In addition, when the imaging device 21 of the scanning device 20 obtains the image of the oral cavity, the scanning device 20 also through the orientation sensor 22 obtains the orientation data of the imaging device 21 when capturing the image. These orientation data are related to the positions and angles of the teeth shown in FIG. 3, as well as the front/inside and chewing surfaces of the teeth.

After receiving the oral cavity image, the controller 23 uses a zone classifier(not shown) to classify (or partition) the oral cavity image according to the oral cavity image and/or the orientation data. For example, classify the oral cavity images to different regions; such as the "upper right quadrant", "upper left quadrant ", "lower right quadrant", "lower left quadrant" in FIG. 3.

The function performed by the zone classifier(not shown) is implemented, for example, by the controller 23 executing a deep learning zone classification model that has completed training. However, it is not limited to this deep learning zone classification model, and may also be a simple computer graphics recognition program. Here, the deep learning zone classification model is trained by at least the images of oral cavity. Such images have been pre-labeled the "upper right quadrant", "upper left quadrant", "lower right quadrant ", "lower left quadrant", and/or the front, inside or chewing surfaces of teeth. After completing the training, the deep learning zone classification model extracts the features of the received image (for example, the location (quadrant) and tooth surface region), and uses the extracted features for zone classification. Here, the controller 23 performs the function of the zone classifier to classify the image to a large region (that is coarse classification for the image) in advance.

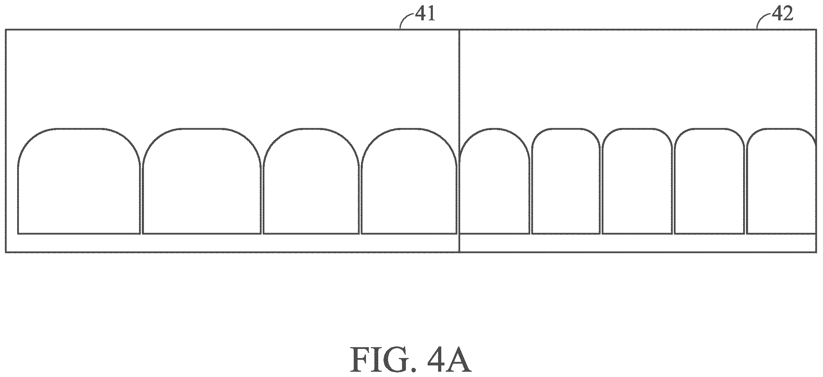

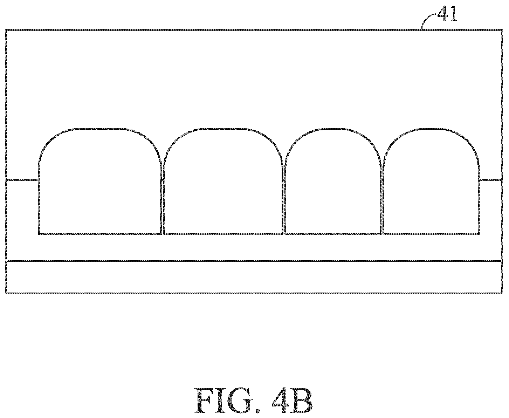

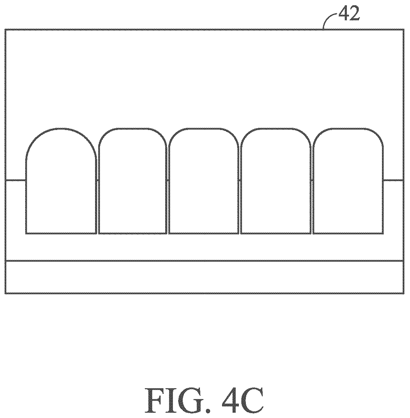

FIG. 4A shows an exemplary image of teeth in oral cavity. Fig. 4B shows the image 41 classified to the lower region in FIG. 3. The image 41 is, for example, a front image of four incisor teeth (here, tooth numbers 42, 41, 32, 31). Fig. 4C shows the image 42 classified to the lower region in FIG. 3. The image 42 is, for example, a front image of one canine tooth (tooth number 33) and four incisor teeth (tooth numbers 34, 35, 36, 37).

Then, the controller 23 uses an object detector (not shown) to detect each object in the different regions. Here, the controller 23 executes the deep learning detection model that has completed training to realize the function of the object detector to perform object detection. Here, the deep learning detection model is trained using learning data that has been pre-labeled with attributes such as object type, location, size, and range. The deep learning detection model after completion of training can identify the type of object (such as tooth type, gums, plaque, etc.), the position of the objects relative to each other, the size (length and width), and the range of the object, etc.

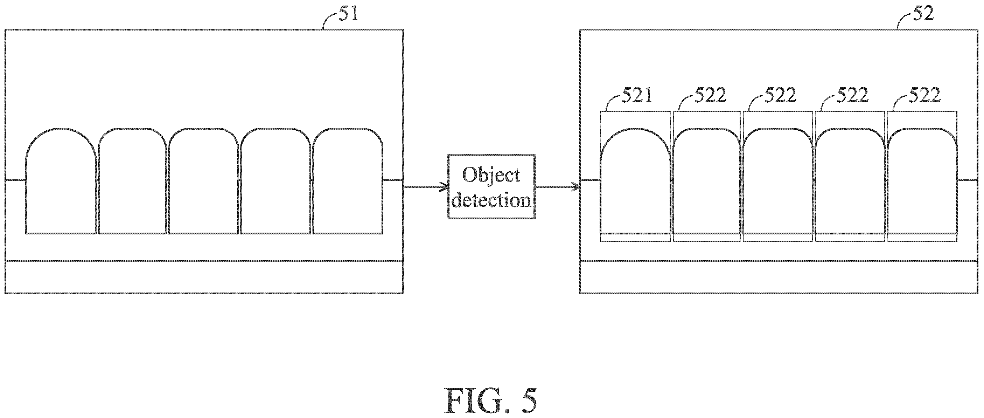

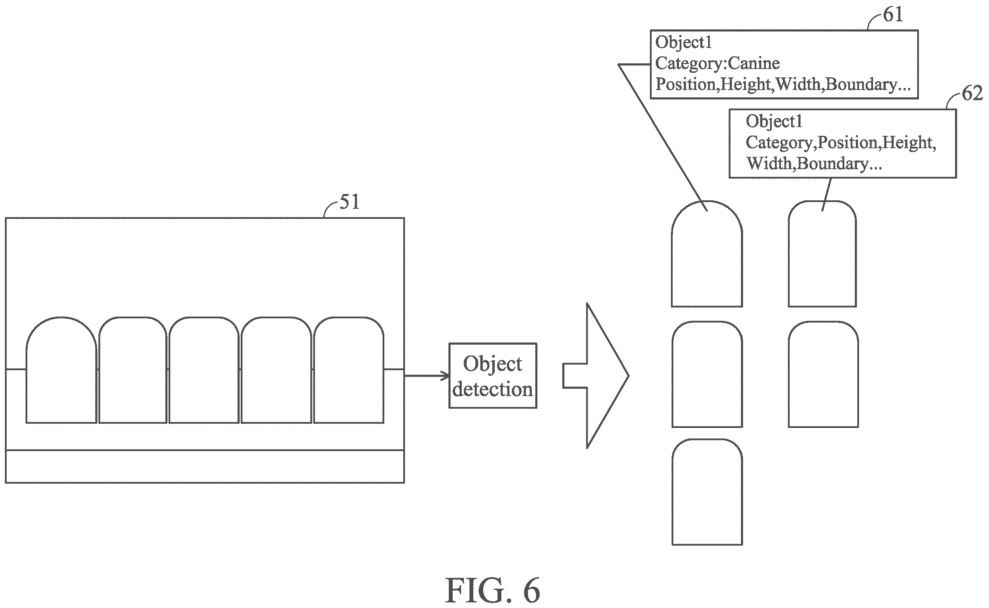

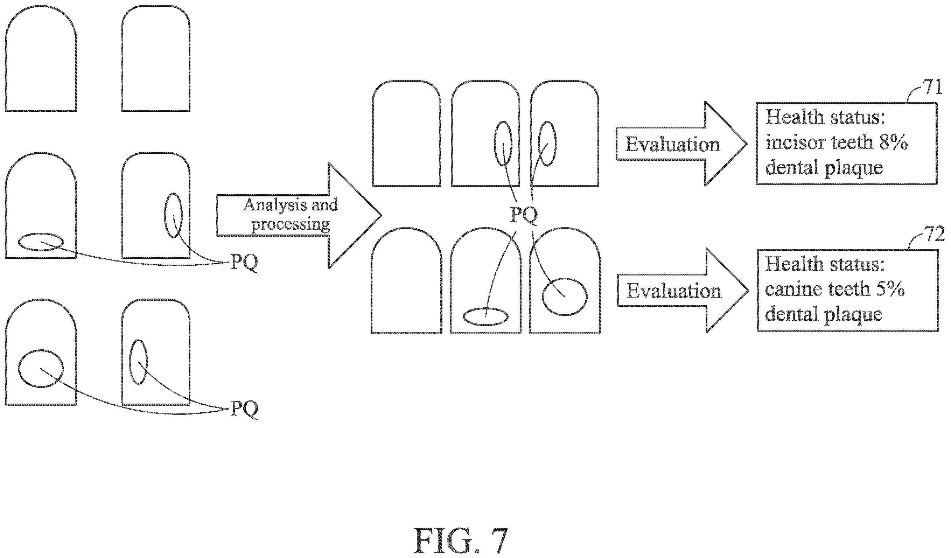

FIG. 5 is a schematic diagram showing the execution of object detection according to the present invention. FIG. 6 shows a schematic diagram of annotating properties with text after executing the object detection. FIG. 7 shows a schematic diagram of identifying canine teeth, incisor teeth and dental plaque during object detection. The controller 23 executes the function of the object detector, and after detecting the oral cavity image 51, canine teeth 521 and incisor teeth 522 can be automatically framed in the image 52, as shown in FIG. 5. In addition, the types of teeth (canine or incisor teeth), the length and width of the teeth, and the inclusive range and boundary of teeth may be further annotated with text, as shown in blocks 61 and 62 in FIG 6.

The deep learning detection model is executed in the controller 23, and the detected objects can be further distinguished as teeth, dental plaques or gums. Referring to FIG. 7, for example, in the detection of objects, in addition to identifying canine teeth and incisor teeth, dental plaques PQ on canine teeth and incisor teeth can also be further identified.

Then, the controller 23 analyzes the dental plaques on the canine teeth and the incisor teeth in FIG. 7 through the processing model for evaluating the status of the teeth. The processing model here is, for example, a model specially used for treating dental plaques.

The processing model evaluates the health status based on the detected dental plaques PQ in the canine teeth and incisor teeth respectively. Here, the processing model can, for example, calculate the area percentage occupied by dental plaque on the surfaces of canine teeth and incisor teeth, and evaluate the health status of the teeth based thereon. For example, the annotation 71 in FIG. 7 indicates that the plaque percentage on the incisor teeth is 8%, while the annotation 72 in FIG. 7 indicates that the plaque percentage on the canine teeth is 5%. Finally, the controller 23 integrates and outputs the status evaluation results of the processing model.

### [Third embodiment]

According to the third embodiment of the present invention, a health status evaluation method is proposed, which can be applied to the scanning device described above, such as the body scanner of the first embodiment or the oral scanner of the second embodiment. In this embodiment, body-part images are, for example, facial images, hand images, or other images at different body locations, and of course also include the oral cavity images in the second embodiment.

The health status evaluation method of this embodiment includes the following steps. First, a body-part image is obtained from the imaging device of the scanning device. Next, the controller of the scanning device classifies (groups) the body-part image in advance to an appropriate category. Then, a processing model suitable for evaluating the body-part images is selected based on the classification result performed by the controller. Next, the controller uses the selected processing model to perform status evaluation on the body part. Finally, the controller integrates and outputs the status evaluation result of the processing model.

It should be noted that before the controller uses the selected processing model to evaluate the status of the body-part image, the controller further performs the following steps. First, the controller further obtains the orientation data from the orientation sensor of the imaging device. Second, the controller performs the function of zone classification according to at least one of the characteristics of the body-part image and orientation data to further classify different objects in the body-part image to multiple different regions or groups. Third, the controller performs an object detection function to detect each of the objects classified to different regions. Then, the controller uses the selected processing model to perform status evaluation on each of the objects detected in the different regions, and integrate and output the evaluation results.

In addition, the controller performs the function of zone classification by executing the deep learning zone classification model that has completed training to classify objects. Moreover, the aforementioned deep learning zone classification model is trained using body-part image learning data that have been pre-labeled with orientation data.

In addition, when the controller performs the object detection function, it executes the deep learning detection model that has completed training to perform object detection. Moreover, the aforementioned detection model is trained using learning data that has been pre-labeled with attributes such as object type, location, size, and range.

It can be seen from the above embodiments, according to the health status evaluation method of the present invention and the scanning device using the sane, body parts are first pre-grouped, and then an appropriate model is automatically selected to evaluate the body parts according to the classification results, and finally all the evaluation results are integrated and an overall result report is given. In this manner, medical personnel can assess health status more quickly and accurately. Furthermore, through automated object classification and model selection, faster decisions and more accurate responses can be achieved.

While the invention has been described by way of example and in terms of the preferred embodiments, it should be understood that the invention is not limited to the disclosed embodiments. On the contrary, it is intended to cover various modifications and similar arrangements (as would be apparent to those skilled in the art). Therefore, the scope of the appended claims should be accorded the broadest interpretation so as to encompass all such modifications and similar arrangements.

## Claims

1. A health status evaluation method, comprising:
obtaining a body-part image from an imaging device;
classifying the body-part image;
selecting a processing model suitable for evaluating the body-part image based on the classification result;
using the selected processing model to perform health status evaluation on the body part corresponding to the body-part image; and
integrating and outputting the health status evaluation result of the processing model.

2. The health status evaluation method as claimed in claim 1, wherein before using the processing model to evaluate the status of the body part, the method further comprises:
receiving orientation data obtained by an orientation sensor of the imaging device;
using a zone classifier to further classify different objects in the body-part image to multiple different regions based on at least one of the characteristics of the body-part image and the orientation data; and
using an object detector to detect each object in the different regions;
afterwards, using the processing model to perform the health status evaluation on each of the objects detected in the different regions.

3. The health status evaluation method as claimed in claim 2, wherein the zone classifier performs object zone classification by executing a deep learning zone classification model that has completed training; and the object detector performs object detection by executing a deep learning detection model that has completed training.

4. A scanning device, comprising:
an imaging device, configured to obtain a body-part image;
an orientation sensor, configured to obtain orientation data related to the imaging device;
a controller, configured to execute the steps of:
receiving the body-part image from the imaging device;
classifying the body-part image;
selecting a processing model suitable for evaluating the body-part image based on the classification result;
using the selected processing model to perform health status evaluation on the body part corresponding to the body-part image; and
integrating and outputting the health status evaluation result of the processing model.

5. The scanning device as claimed in claim 4, wherein the controller classifies the body-part image by using an image classification device, or using a zone classifier by executing a deep learning zone classification model that has completed training.

6. The scanning device as claimed in claim 5, wherein before using the processing model to evaluate the status of the body part, the controller further executes the steps of:
receiving the orientation data output by the orientation sensor;
using the zone classifier to further classify different objects in the body-part image to multiple different regions based on at least one of the characteristics of the body-part image and the orientation data; and
using an object detector to detect each object in the different regions;
afterwards, using the processing model to perform the health status evaluation on each of the objects detected in the different regions.

7. The scanning device as claimed in claim 6, wherein the controller executes a deep learning zone classification model that has completed training to perform zone classification.

8. The scanning device as claimed in claim 7, wherein the deep learning classification model is trained using body-part image learning data that has been pre-labeled with orientation data.

9. The scanning device as claimed in claim 6, wherein the controller executes the trained deep learning detection model to realize the object detector for performing object detection.

10. The scanning device as claimed in claim 9, wherein the deep learning detection model is trained using learning data that has been pre-labeled with attributes of object type, location, size, and range.
